# EUROPÄISCHE PATENTSCHRIFT

(11) **EP 2 717 969 B1**
(45) Veröffentlichungstag und Bekanntmachung des Hinweises auf die Patenterteilung: **07.11.2018**
(21) Anmeldenummer: 12727304.3
(22) Anmeldetag: 05.06.2012
(51) Int. Cl.: A61Q 19/00, A61Q 17/04, A61K 8/04, A61K 8/06, A61K 8/37, A61K 8/39, A61K 8/34, A61K 8/41, A61K 8/44, A61K 8/49, A61Q 17/00

(54) **KOSMETISCHE ODER DERMATOLOGISCHE ZUBEREITUNGEN MIT VERBESSERTER MIKROBIOLOGISCHER STABILITÄT**
COSMETIC OR DERMATOLOGICAL PREPARATIONS WITH IMPROVED MICROBIOLOGICAL STABILITY
COMPOSITIONS COSMÉTIQUES OU DERMATOLOGIQUES AYANT UNE STABILITÉ MICROBIOLOGIQUE AMÉLIORÉE

(30) Priorität: 07.06.2011 DE 102011077045
(43) Veröffentlichungstag der Anmeldung: 16.04.2014
(73) Patentinhaber: Beiersdorf AG, 20253 Hamburg (DE)
(72) Erfinder: VON THADEN, Stefanie, 20255 Hamburg (DE); KÖHLER, Manuela, 22147 Hamburg (DE)
(74) Vertreter: Hartmann, Jost
(86) Internationale Anmeldenummer: PCT/EP2012/002371
(87) Internationale Veröffentlichungsnummer: WO 2012/167902

(56) Entgegenhaltungen:
- EP-A1- 1 752 193
- EP-A1- 2 623 092
- WO-A1-2012/019789
- WO-A2-2010/110579
- WO-A2-2011/124241
- WO-A2-2011/124245
- DE-A1- 19 547 679
- JP-A- 2002 322 021
- DATABASE WPI Week 200453 Thomson Scientific, London, GB; AN 2004-546800 XP002716806, & JP 2004 196666 A (POLA CHEM IND INC) 15. Juli 2004 (2004-07-15)
- "Product Information: dermosoft lp", , 1. Januar 2010 (2010-01-01), Seiten 1-11, XP055079646, Hamburg, Germany Gefunden im Internet: URL:http://www.dr-straetmans.de/customer-l ogin/productdocumentation/Dermosoft_LP/Pro duct_Leaflet_Dermosoft_LP.pdf [gefunden am 2013-09-17]
- "Polyglyceryl-10-Stearate - INCI Monograph ID: 4140 ED - Gottschalck T E; Bailey (Editors) J E", 1. Januar 2009 (2009-01-01), INTERNATIONAL COSMETIC INGREDIENT DICTIONARY AND HANDBOOK, PERSONAL CARE PRODUCTS COUNCIL, WASHINGTON D.C. (US), PAGE(S) 2358, XP002693294, das ganze Dokument

## Beschreibung

Die Erfindung umfasst kosmetische oder dermatologische Zubereitungen mit Polyglyceryl-10-Stearat mit verbesserter mikrobiologischer Stabilität und Hautverträglichkeit.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind am weitesten verbreitet wiederum die eigentlichen Emulsionen.

Aber auch emulgatorarme bzw. -freie Zubereitungen auf Basis sogenannter Hydrodispersionen sind bekannt. Hydrodispersionen stellen Dispersionen einer flüssigen, halbfesten oder festen inneren (diskontinuierlichen) Lipidphase in einer äußeren wäßrigen (kontinuierlichen) Phase dar. Hydrodispersionen sind - wie auch Emulsionen, die sich durch eine ähnliche Phasenanordnung auszeichnen - metastabile Systeme und daher geneigt, in einen Zustand zweier in sich zusammenhängender diskreter Phasen überzugehen. In einer klassischen O/W-Emulsion verhindert die Wahl eines geeigneten Emulgators die Phasentrennung. Im Gegensatz zur klassischen Emulsionen enthalten Hydrodispersionen aber nur sehr geringe Mengen an Emulgatoren bzw. können sogar gänzlich frei von Emulgatoren sein.

Als kosmetische oder medizinische Zubereitungen sind oftmals Emulsionen, insbesondere W/O-, O/W-, O/W/O oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Der Stand der Technik kennt mehrere wesentliche Faktoren, die einen positiven Einfluss auf die Stabilität und Rheologie von Emulsionen haben.

Emulsionen benötigen zu ihrer Bildung und zur Stabilisierung im Allgemeinen einen oder mehrere Emulgatoren, Verdicker und/oder Konsistenzgeber, um über einen kosmetisch akzeptablen Zeitraum stabil zu sein, im Allgemeinen 1 Jahr nach dem Öffnen einer kosmetischen Zubereitung.

Dazu werden oft große Mengen an Emulgatoren benötigt. Dies wiederum kann bei Konsumenten zu Missempfindungen bis zur Unverträglichkeit und im Extremfall sogar zu Phänomenen wie die "Mallorca Akne" o.ä. führen.

Wünschenswert ist es daher Emulsionszubereitungen zur Verfügung zu stellen, die möglichst geringe Mengen an Emulgatoren umfassen und dennoch ausreichend stabil formuliert sind.

Damit kosmetische oder dermatologische Cremes und Lotionen nach dem Öffnen noch einige Monate haltbar sind, müssen sie konserviert werden. Entscheidend ist die Wahl der Mittel - denn Bakterien, Pilze und Hefen unterscheiden nicht, ob eine Creme oder Lotion natürliche oder synthetische Stoffe enthält. Einmal öffnen genügt und schon ist die kosmetischen Zubereitung mit Bakterien, Pilzen und/oder Hefen kontaminiert und können sich vermehren. Die Verkeimung von Kosmetika gilt es aus zwei Gründen zu vermeiden. Zum Einen muss die Formel vor eventuellen Abbau von Rohstoffen geschützt werden. Der Abbau von Rohstoffen kann zu den verschiedensten Veränderungen der Formel führen, zum Beispiel Instabilität (wenn z.B. der Emulgator abgebaut wird). Aber auch vor den eventuellen Abbauprodukten der Mikroorganismen muss die Formel geschützt werden (Duft- oder Farbveränderung etc.). Zu guter Letzt können Mikroorganismen Toxine bilden.

Gemäß der Council Directive 76/768 EEC dürfen ausschließlich sichere kosmetische Mittel vermarktet werden. Das heißt auch, dass kosmetische Mittel, die aufgrund ihrer chemischen Zusammensetzung und physikalischen Eigenschaften das Wachstum von Mikroorganismen zulassen, konserviert werden müssen. Hierzu sind die in Annex VI der Council Directive 76/768 aufgeführten und als sicher bewerteten Konservierungsmittel einzusetzen, um den Anwender vor durch Mikroorganismen ausgelöste Gesundheitsgefährdungen zu schützen und durch das Wachstum von Mikroorganismen verursachte Produktschädigungen (Abbau oder Veränderungen von Inhaltsstoffen, mikrobielle Stoffwechselendprodukte) abzuwenden. Damit wird die mikrobiologische Stabilität des kosmetischen Mittels während des Produktlebenszyklus (Produktion, Lagerung, Anwendung) gewährleistet. Die Konzentration von Konservierungsmitteln sollte so gewählt werden, dass diese Ziele erreicht werden, eine Überkonservierung aber vermieden wird. Hierdurch wird für den Anwender das nicht vollständig zu vermeidende Restrisiko von unerwünschten Nebenwirkungen (Hautirritation, Sensibilisierung) zusätzlich minimiert. Die Reduktion an Konservierungsmitteln gelingt jedoch nur, wenn Inhaltsstoffe kosmetischer Mittel zusätzlich als Sekundäreigenschaft antimikrobielle Eigenschaften aufweisen.

Konservierungsstoffe haben den Auftrag, Bakterien, Hefen und Pilzen abzutöten. Viele dieser Stoffe sind sehr reaktiv und haben cytotoxische Wirkungen, die auf der Haut zu Irritationen und Rötungen führen können. Bei langjähriger Anwendung besteht zudem ein Restrisiko für besonders empfindliche Verbraucher. Bei langjähriger Anwendung besteht zu dem die Gefahr, dass die Stoffe den Organismus sensibilisieren und es zu allergischen Reaktionen kommt. Natürlich ist die Empfindlichkeit von vielen Parametern abhängig: Konzentration, Expositionsrate, Art der Substanz, Löslichkeitsverhalten, Ort der Anwendung und individueller Vorbelastung.

Bekannte, wenn gleich vergleichsweise schwache, Allergene sind Formaldehyd und Stoffe, die im Produkt Formaldehyd freisetzen. Auch die halogenorganischen Verbindungen, zu denen einige Konservierungsmittel gehören, haben ein großes allergisches Potenzial.

Kosmetische Zubereitungen müssen jedoch langzeitstabil gegen mikrobielle Kontamination formuliert werden und trotzdem eine sehr gute Hautverträglichkeit aufweisen.

Die mikrobielle Stabilität wird bislang durch den Zusatz an Konservierungsmitteln gelöst. Bekannte Konservierungsmittel sind die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben.

Des Weiteren ist Phenoxyethanol, Ethylenglycolmonophenylether, Phenylglycol, Phenoxetol®, Benzoesäure, Benzoate, Formiate und Teebaumöle zur Konservierung von kosmetischen Mitteln bekannt.

Wünschenswert ist es daher die Menge an Konservierungsmitteln in kosmetischen oder dermatologischen Zubereitungen weitest möglich zu reduzieren.

Hinsichtlich zunehmender Gesundheitsbeeinträchtigungen und Allergien sowie dem Drang der Konsumenten nach mehr ökologisch verträglichen Kosmetika besteht das Problem, dass diese Zubereitungen dennoch eine ausreichende Stabilität und vor allem Hautverträglichkeit aufweisen müssen.

Des Weiteren ist ein einfacher Austausch oder das Vermeiden von Konservierungsstoffen wie Parabene und/oder Phenoxyethanol ohne Einbußen hinsichtlich der Stabilität und Anwendungseigenschaft nicht ohne weiteres möglich.

Aus der Fachliteratur (Preservatives for Pharmaceuticals" J.Soc. Cosmet. Chem. 23 703-720 1972) ist bekannt, dass die Wahl der Rezepturbestandteile und Formulierungen die Verfügbarkeit von enthaltenen antimikrobiellen Substanzen beeinflussen kann. So können zum Beispiel hohe Emulgatorgehalte die Effektivität und Wirksamkeit von antimikrobiellen Substanzen herabsetzen. Die Verfügbarkeit antimikrobieller Moleküle kann unter anderem durch Komplexierung mit Emulgatoren herabgesetzt werden. Die Menge an gebundenem antimikrobiellen Wirkstoff steht hiernach in direktem Zusammenhang mit der eingesetzten Emulgatormenge.

Wünschenswert ist es daher Zubereitungen zur Verfügung zu stellen, die möglichst geringe Mengen an Konservierungsmitteln umfassen und dennoch ausreichend stabil formuliert sind und insbesondere eine ausreichende mikrobiologische Stabilität aufweisen.

Weiterhin müssen kosmetische oder dermatologische Zubereitungen einigen ästhetischen Gesichtspunkten genügen um eine ausreichende Verbraucherakzeptanz zu erlangen.

Dem Fachmann sind Emulgatoren aus dem Lebensmittelbereich bekannt wie Glyceryl Stearate Citrat (Imwitor®) oder Polyglyceryl-10-Stearat.

Auf der Suche nach geeigneten Emulgatoren, die den gewünschten Anforderungen der Kosmetik oder Dermatologie entsprechen, scheinen diese Emulgatoren sich aber auszuschließen.

Glyceryl Stearate Citrat muss in kosmetischen Zubereitungen in einer solch hohen Menge oder in Kombination mit Co-Emulgatoren eingesetzt werden um stabile Emulsionen zu erhalten, so dass der Wunsch nach verringertem Emulgatorgehalt mit Glyceryl Stearate Citrat nicht zu erfüllen ist.

Polyglyceryl-10-Stearat (PG-10-Stearat, CAS-Nr.: 79777-30-3) ist ein hydrophiler Emulgator und weist einen HLB von 12 auf.

Im Handel sind Polyglyceryl-10-Stearate als Mischungen beispielsweise erhältlich als Polyaldo 10-1-S (Lonza), Nikkol Decaglyn 1-S (Nikko Chemicals Co., Ltd.) oder Salacos PGMSV (The Nisshin OilliO Group, Ltd.).

Polyglyceryl-10-Stearat ist aus der Eiscremeherstellung bekannt und weist eine braune Farbe auf. Auch aufgrund dieses ästhetisch problematischen Umstandes schien sich die Anwendung in kosmetischen Emulsionen von selbst zu verbieten.

Käuflich erwerbar ist Heliogel®, welches neben Sodium Acrylates Copolymer, Hydrogenated Polyisobutene, Phospholipids, Helianthus Annuus (Sunflower) Seed Oil auch Polyglyceryl-10-Stearate umfasst.

In kosmetischen Zubereitungen ist PG-10-Stearat lediglich als Bestandteil eines Aftershavegels genannt. In dieser Zubereitung sind 6 Gew.% PG-10-Stearat neben weiteren Emulgatoren wie Lecithin und PEG 150 distearat beschrieben (WO 2003082182).

JP 2005162664 offenbart PG-10-stearat in Haarfärbezubereitung umfassend Peroxide. Eine kosmetische topische Applikation schließt sich daher aus.

US 5925615 offenbart PG-10-stearat in Shampooformulierungen umfassend Polyethylenglykole und Parabene.

DE 19547679 A1 beschreibt eine Haarpflegeemulsionen mit PG-10-stearat und Konservierungsmitteln.

DE 19724587 A1 beschreibt eine Haarspülung mit PG-10-stearat sowie Polyethylenglykole und Parabene.

WO 0027197 A1 offenbart Zubereitungen umfassend neben PG-10-stearat ein oder mehrere UV Filter, Lecithine, Polyethylenglykole und Parabene.

DE 102008028821 A1 und DE 102008028822 A1 offenbaren Stiftzubereitungen mit PG-10-stearat.

DE 10213955 A1 offenbart PG-10-stearat in einer After Shave Lotion, wobei der Gehalt an PG-10-stearat 6% und mehr beträgt.

US 20060018853 beschreibt peel-off Zubereitung mit PG-10-stearat mit Decaglyceryltriisostearat und Lecithin.

JP 2004 196666 A beschreibt eine kosmetische Zusammensetzung für empfindliche Haut, welche 1,5% PG-10-stearat und Phenoxyethanol als antimikrobiellen Stoff enthält.

Überraschenderweise zeigte sich, dass sich Polyglyceryl-10-Stearat in kosmetischen oder dermatologischen Zubereitungen als Emulgator einsetzen lässt, ohne die Nachteile der färbenden Wirkung und der Instabilität zu zeigen.

Überraschenderweise erweist sich Polyglycerin-10-Stearat auch als effizienter Zusatz um die Menge an Konservierungsmitteln zu reduzieren.

Die Erfindung ist eine kosmetische oder dermatologische Zubereitung umfassend Polyglyceryl-10-Stearat mit weniger als 6 Gew.%, insbesondere im Bereich von 0,1 bis 2,8 Gew.%, bezogen auf die Gesamtmasse der Zubereitung, und einen Anteil an antimikrobiell wirkenden Substanzen (Konservierungsmitteln) im Bereich von mindestens 0,05 Gew.% und maximal 5 Gew.%, bezogen auf die Gesamtmasse der Zubereitung.

Die Zubereitung ist frei von Lecithinen, Parabenen, Formaldehydabspaltern, organischen Halogeniden, ausgenommen Benzethoniumchlorid, Benzoesäure und ihren Salzen, Formiaten und Teebaumöl. Als antimikrobielle Stoffe werden Piroctone Olamine, Methylisothiazolinone, Phenethylethanol und/oder Benzethoniumchlorid gewählt.

Ausgenommen von den erfindungsgemäßen Zubereitungen sind O/W-Emulsionszubereitungen 1, 3, 4 und 5 bestehend aus

| | 1 | 3 | 4 | 5 |
|---|---|---|---|---|
| Panthenol | 1,5 | 0 | 0,7 | 0 |
| Butylenglykol | --- | --- | 5 | 10 |
| Propylenglykol | --- | 10 | 5 | --- |
| Benzethoniumchlorid | 1,0 | 0,25 | 0,5 | 0,9 |
| Lauroylethylarginat | 1,0 | 1,5 | 1,0 | 0,5 |
| medizinisches Weissöl | 0 | 6 | 0 | 0 |
| Isopropylpalmitat | 1 | 0 | 0 | 0 |
| Caprylic/Capric Triglycerid | 0 | 3 | 0 | 1,00 |
| Cetearylalkohol | 0 | 0 | 2,5 | 4,00 |
| Cetylalkohol | 0 | 3 | 0 | 0 |
| lineares Silikonöl | 0 | 3 | 0 | 0,50 |
| cyclisches Silikonöl | 0 | 3 | 0 | 0 |
| C12-15 Alkylbenzoat | 0 | 0 | 0 | 2,00 |
| Sheabutter | 0 | 0,5 | 0 | 0 |
| Dicaprylylether | 0 | 0 | 10 | 0 |
| Dicaprylylcarbonat | 2 | 0 | 0 | 0 |
| Glyceryl stearat | 0 | 1,2 | 2,4 | 2,4 |
| Tapiocastärke | 1 | 0 | 0 | 0 |
| Glycerin | 5 | 10 | 8 | 8 |
| Butylenglykol | 0 | 0 | 1 | 0 |
| Zitronensäure | 0,0050 | 0 | 0 | 0 |
| Natronlauge 45% | 0,35 | 0,01 | 0,25 | 1 |
| Polyglyceryl-10 Myristat | 0 | 0 | 0 | 0 |
| Polyglyceryl-10 Stearat | 0,7 | 1,8 | 1 | 1 |
| Polyacrylsäure, Na-Salz (Carbopol 981) | 0,5 | 0,02 | 0 | 0 |
| Acrylic Acid/VP Crosspolymer | 0 | 0 | 0,5 | 0,75 |
| Trinatrium EDTA | 0 | 1 | | 1 |
| Ethylhexylmethoxycinnamat | 0 | 0 | 2 | 0 |
| Butylmethoxydibenzoylmethan | 0 | 0 | 0 | 2 |
| Phenylbenzimidazolesulfonsäure, Natrium-Salz | 0 | 0 | 0 | 2 |
| Ethylhexylsalicylat | 0 | 0 | 0 | 2 |
| Titandioxid, silikongecoated | 0 | 0 | 0,3600 | 0 |
| Octocrylen | 0 | 0 | 0 | 2 |
| Parfum | 0,10 | 0,35 | 0 | 0,20 |
| Wasser | ad 100 | ad 100 | ad 100 | ad 100 |

sowie O/W-Emulsionszubereitungen 46, 48, 49 und 50 bestehend aus

| | 46 | 48 | 49 | 50 |
|---|---|---|---|---|
| Panthenol | 1,5 | 0 | 0,7 | 0 |
| Butylenglykol | --- | --- | 5 | 10 |
| Propylenglykol | --- | 10 | 5 | --- |
| Methylisothiazolinone | --- | --- | 0,06 | --- |
| Benzethoniumchlorid | 2,0 | --- | 0,15 | --- |
| Piroctonolamin | --- | 0,25 | 0,15 | --- |
| Lauroylethylarginat | --- | --- | 0,15 | 1,75 |
| medizinisches Weißöl | 0 | 3 | 0 | 0 |
| Isopropylpalmitat | 1 | 0 | 0 | 0 |
| Caprylic/Capric Triglycerid | 0 | 3 | 0 | 1,00 |
| Cetearylalkohol | 0 | 0 | 2,5 | 4,00 |
| Cetylalkohol | 0 | 3 | 0 | 0 |
| lineares Silikonöl | 0 | 3 | 0 | 0,50 |
| zyklisches Silikonöl | 0 | 3 | 0 | 0 |
| C12-15 Alkylbenzoat | 0 | 0 | 0 | 2,00 |
| Sheabutter | 0 | 0,5 | 0 | 0 |
| Dicaprylylether | 0 | 0 | 10 | 0 |
| Dicaprylylcarbonat | 2 | 0 | 0 | 0 |
| Glyceryl Stearate | 0 | 1,2 | 2,4 | 2,4 |
| Tapiocastärke | 1 | 0 | 0 | 0 |
| Glycerin | 5 | 10 | 8 | 8 |
| Zitronensäure | 0,0050 | 0 | 0 | 0 |
| Natronlauge 45%ig | 0,35 | 0,01 | 0,25 | 1 |
| Polyglyceryl-10 Myristat | 0 | 0 | 0 | 0 |
| Polyglyceryl-10 Stearat | 0,7 | 1,8 | 1 | 1 |
| Polyacrylsäure, Na-Salz | 0,5 | 0,02 | 0 | 0 |
| Acrylic Acid/VP Crosspolymer | 0 | 0 | 0,5 | 0,75 |
| Trinatrium EDTA | 0 | 1 | | 1 |
| Ethylhexylmethoxycinnamat | 0 | 0 | 2 | 0 |
| Butylmethoxydibenzoylmethan | 0 | 0 | 0 | 2 |
| Phenylbenzimidazolesulfonsäure | 0 | 0 | 0 | 2 |
| Ethylhexylsalicylat | 0 | 0 | 0 | 2 |
| Titandioxid+ Trimethoxycaprylylsilan | 0 | 0 | 0,3600 | 0 |
| Octocrylen | 0 | 0 | 0 | 2 |
| Parfum | 0,10 | 0,35 | 0 | 0,20 |
| Wasser | ad 100 | ad 100 | ad 100 | ad 100 |

Die Zahlenwerte stellen Gewichtsanteile bezogen auf die Gesamtmasse der Zubereitungen dar.

Besonders vorteilhaft hinsichtlich Verbraucherakzeptanz und Hautverträglichkeit umfasst die erfindungsgemäße Zubereitung keinen Alkohol, insbesondere kein Ethanol.

Ethanol wird insbesondere bei Konsumenten mit empfindlicher Haut als reizend bis brennend empfunden und damit abgelehnt.

Die erfindungsgemäßen Zubereitungen basieren vorteilhaft auf einer Emulsion, bevorzugt einer O/W-Emulsion, oder Hydrodispersion. Die so hergestellten Zubereitungen, bevorzugt Emulsionen, weisen eine niedrigen Emulgatorgehalt von weniger als 6% auf und erfüllen somit den Wunsch nach gut verträglichen, milden Produkten, ohne das die Stabilität beeinträchtigt wird. Weniger als 6 Gew.% bzw. weniger als 3 Gew.% PG-10-Stearat bedeutet nicht 0 Gew.%. Bevorzugt beträgt der Mindestanteil an PG-10-Stearat 0,1 Gew.%.

Überraschenderweise sind die mit weniger als 6 Gew.% Polyglyceryl-10-Stearat hergestellten Zubereitungen stabil für einen Zeitraum von mindestens 3 Jahre bei Raumtemperatur und mindestens 6 Monate bei 40°C Lagerung.

Des Weiteren zeigte sich überraschend, dass Polyglyceryl-10-Stearat, auch als einziger Emulgator eingesetzt, zu stabilen Emulsionen führt. Ein Verzicht auf weitere zusätzliche Emulgatoren ist damit möglich und kommt so dem Wunsch nach verringerten Emulgatorgehalten nach.

Emulgatoren haben bekanntermaßen keinen oder nur sehr geringen Einfluss auf die mikrobiologische Stabilität, ausser zuvor erwähnte Komplexierung enthaltener Konservierungsmittel.

Zubereitungen umfassend PG-10-Stearat zeigen überraschenderweise eine bessere mikrobiologische Stabilität durch den Einsatz von PG-10-Stearat im Vergleich zu anderen Emulgatoren, insbesondere anstelle von PEG-40-Stearat, auch wenn der Anteil an Konservierungsmittel reduziert wird.

Polyglyceryl-10-Stearat kann daher als Emulgator der Wahl in kosmetischen oder dermatologischen Zubereitungen zur Verbesserung der mikrobiologischen Stabilität einer kosmetischen Formel verwendet werden.

Der Nachweis der mikrobiologischen Stabilität eines kosmetischen Mittels wird in der Regel mit der Prüfung auf ausreichende Konservierung (Konservierungsbelastungstest) nachgewiesen. Hierbei werden Muster eines zu untersuchenden kosmetischen Mittels mit Testkeimen (Anfangskeimzahl 1E+5 Koloniebildende Einheiten pro g) kontaminiert und anschließend die Lebendzellzahl in definierten Zeitintervallen bestimmt. Aus dem hieraus zu berechnenden logarithmischen Reduktionsfaktoren (log RF) lässt sich die Wirksamkeit des Konservierungsmittels bestimmen.

Bei einer vergleichenden Untersuchung eines kosmetischen Mittels (siehe Tabelle 1) basierend auf 0,8% PEG-40-Stearat bzw. 0,8% PG-10-Stearat zeigte sich im Konservierungsbelastungstest bei vergleichbarer konservierender Wirkung gegen Staphylococcen, Hefen und Schimmelpilze eine bessere Inaktivierungskinetik gegenüber Pseudomonaden (Tabelle 2 und 3). Während in dem kosmetischen Mittel mit 0,8% PEG-40-stearat nach 7d noch 1,8E+4 Koloniebildenden Einheiten pro g dieser Keimgruppe nachweisbar waren (log RF 1), lag die in dem mit 0,8% PG-10-stearat nach 7d ermittelte Keimzahl für Pseudomonaden mit <1E+2 Koloniebildenden Einheiten pro g (log RF >3) deutlich niedriger.

Dieses läßt auf eine wesentlich stärkere antibakterielle Wirkung gegen Pseudomonaden schließen, wodurch auf den Einsatz weiterer Konservierungsmittel verzichtet werden kann ohne die sichere Verwendung des kosmetischen Mittels zu gefährden.

**Tabelle 1:**

| INCI | 1 | 2 |
|---|---|---|
| Cetearyl Alcohol | 3.0000 | 3.0000 |
| Caprylic/Capric Triglyceride | 2.5000 | 2.5000 |
| Dicaprylyl Carbonate | 2.5000 | 2.5000 |
| Dimethicone | 0.3500 | 0.3500 |
| C12-15 Alkyl Benzoate | 2.5000 | 2.5000 |
| Cyclomethicone | 2.1500 | 2.1500 |
| PEG-40 Stearate | 0.8000 | - |
| Glyceryl Stearate | 2.6000 | 2.6000 |
| Nylon-12 | 1.5000 | 1.5000 |
| Tapioca Starch + Aqua | 1.5000 | 1.5000 |
| Parfum | 0.2500 | 0.2500 |
| Glycerin | 8.6500 | 8.6500 |
| Sodium Hydroxide aq | 0.0540 | 0.0540 |
| Polyglyceryl-10-Stearat | - | 0.8000 |
| Benzethonium Chloride | 0.0500 | 0.0500 |
| Methylpropanediol | 4.0000 | 4.0000 |
| Carbomer | 0.1000 | 0.1000 |
| Aqua | 62.4960 | 62.4960 |
| Aqua + Trisodium EDTA | 1.0000 | 1.0000 |
| Ethylhexyl Methoxycinnamate + BHT | 3.0000 | 3.0000 |
| Butyl Methoxydibenzoylmethane | 1.0000 | 1.0000 |

**Tabelle 2:**

| Peg 40 Stear at Basis | Staph aureus/epidermis | Pseudomonas aeruginosa | Pseudomonas putida/cepacia | Enterob. gergovia E coli | Aspergillus brasiliensis | Candida albicans/parapsilosis/guilliermondii |
|---|---|---|---|---|---|---|
| 0d | 2,3E05 | 2,4E05 | 2,5E05 | 2,2E5 | 3,8E05 | 2,3E05 |
| 1d | <10 | <10 | 7,1E04 | <10 | 3,4E04 | 3,7E04 |
| 7d | <10 | <10 | 1,8E04 | <10 | 8,5E02 | <10 |
| 14d | <10 | <10 | <10 | <10 | <10 | <10 |
| 28d | <10 | <10 | <10 | <10 | <10 | <10 |

Die Basis auf PEG 40 Stearat besteht bei allen Keimen die Vollbelastung außer bei dem Pseudomanas putida / cepacia, da hier nach 7 Tagen noch 1,8E04 KBE nachgewiesen werden können.

**Tabelle 3:**

| PG-10-Stearat | Staph aureus/epidermis | Pseudomonas aeruginosa Pseudomonas putida/cepacia Enterob. gergovia E coli | Aspergillus brasiliensis | Candida albicans/parapsilosis/guilliermondii |
|---|---|---|---|---|
| 0d | 2,0E05 | 2,6E05 | 3,5E05 | 3,3E05 |
| 1d | nb | nb | nb | Nb |
| 7d | <10² | <10² | 1,0E01 | <10² |
| 14d | <10² | <10² | <10² | <10² |
| 28d | <10² | <10² | <10² | <10² |

Die Basis auf PG-10-Stearat besteht bei alle Keimen die Vollbelastung, sogar bei den Pseudomonas putida / cepacia nur durch den Wechsel des Emulgators.

Es wurden keine Synergien oder antagonistischen Wechselwirkung zwischen den verschiedenen Konservierungsmittel und PG-10-Stearat beobachtet. Es kann daher davon ausgegangen werden, dass bei der Verwendung von PG-10-Stearat man den Effekt der verbesserten mikrobiologischen Stabilität auch für alle anderen Konservierungsmittel genauso erwarten kann.

D.h. allein durch Zusatz von PG-10-stearat und/oder Ersatz des Emulgatorsystems kann eine deutlich verbesserte mikrobiologische Stabilität erreicht werden ohne die Menge an Konservierungsmitteln erhöhen zu müssen.

Die erfindungsgemäßen Zubereitungen umfassen keine Parabene, Formalgehydabspalter, organohalogenische Stoffe, Benzoesäure und deren Salze, Formiate und Teebaumöl sowie vorteilhaft auch kein Benzylalkohol und/oder Phenoxyethanol.

Diese Substanzen sind allesamt gängige Konservierungsmittel, weisen jedoch auch Nachteile auf. So ist DMDM Hydantoin ein Formaldehydabspalter. Formaldehyd ist wie allgemein bekannt eine giftige Substanz und ihr Gebrauch in Kosmetik nicht unumstritten. Benzylalkohol hat einen charakteristischen Geruch, welche in einer Formulierung mit Parfum maskiert werden muss. Parfuminhaltstoffe sind leider oft auch im Verdacht reizend oder sensibilisierend zu wirken. Deswegen ist es wünschenswert die Parfumkonzentration möglichst gering zu halten. Phenoxyethanol steht immer wieder im Verdacht Typ IV Kontaktallergien auszulösen und wird somit von Konsumenten zunehmend abgelehnt. Organische Halogenide sind vergleichsweise hochreaktive Stoffe und interagieren mit der Zellmembran und werden somit bei den Konsumenten ebenfalls stark abgelehnt. Teebaumöl ist nicht als Arzneimittel zugelassen und wird als Risikosubstanz für das Auftreten von Kontakt-Dermatitiden gewertet.

Die antimikrobiellen Stoffe sind zu wählen aus der Gruppe Piroctone Olamine, Methylisothiazolinone, Phenethylethanol und Benzethoniumchlorid.

Weiterhin zeigte sich überraschenderweise, dass PG-10-Stearat bei der Emulsionsbildung sowohl in die Wasser- als auch in die Fettphase gegeben werden kann. In beiden Fällen wird das gleiche Produkt erhalten. Die Abweichungen in der Konsistenz sind nicht signifikant und für einen Konsumenten nicht relevant.

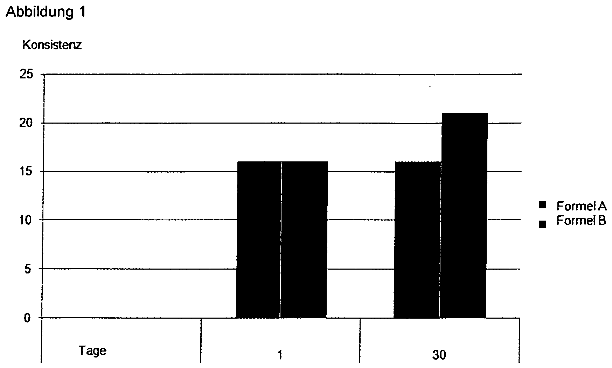

Abbildung 1 zeigt diese Konsistenzuntersuchungen. Die Konsistenz wurde mit einem Konsistenzbestimmungsgerät nach Fligge (gemäß DE 29 09 087) ermittelt.

Auf der X-Achse ist zum Einen der Wert der Konsistenz der zu vergleichenden Formeln A und B nach einem Tag (links) und nach 30 Tagen (rechts) aufgetragen. Die Formel A unterscheidet sich von der Formel B darin, dass der Emulgator PG-10-Stearate im Fall A in die Wasserphase und im Fall B in die Fettphase gegeben wurde. Alle weiteren Schritte waren für beide Formulierungen identisch.

Auf der y- Achse ist der Konsistenzwert aufgetragen. Man erkennt, dass beide Formeln nach 1 Tag sogar numerisch die gleiche Konsistenz haben, also identisch sind bezüglich der Konsistenz. Nach 30 Tagen gibt es zwar einen numerischen Unterschied, der jedoch vom Konsumenten nicht nachvollziehbar ist.

Die Untersuchungen belegen die außergewöhnlichen Eigenschaften von PG-10-Stearat. Da es nicht relevant ist, in welche Phase man den Emulgator gibt, hat man in der Phasenaufteilung in der Produktion größere Flexibilität.

Mit Polyglyceryl-10-Stearat als Emulgator, bevorzugt einzigen Emulgator ist ein Verfahren zur Herstellung kosmetischer oder dermatologischer Emulsionszubereitungen möglich, indem die Emulgatorzugabe sowohl in die Wasser- und/oder in die Ölphase der Emulsion erfolgen kann.

PG-10-sterat haltige Zubereitungen lassen sich somit einfacher herstellen und geben dem Fachmann eine hervorragende Flexibilität bezüglich Viskosität, Sensorik, Applikationsform und Toleranz gegenüber verschiedenster Inhaltsstoffe und der Produktion.

Den Einsatz von PG-10-Stearat in Kosmetika auf möglichst geringe Anteile zu begrenzen mag für einen Kosmetikfachmann aufgrund der braunen Farbe des PG-10-Stearats nahe gelegen haben, jedoch müsste er aufgrund der geringen Anteile dann auch von einer verminderten emulgierenden Wirkung ausgehen. Dies hat sich bei einer Verwendung von weniger als 6 Gew.% PG-10-Stearat, insbesondere im Bereich von 0,1 bis 2,8 Gew.%, bezogen auf die Gesamtmenge der Zubereitung, überraschenderweise aber nicht bewahrheitet.

In mehreren Langzeitstabilitätsuntersuchungen zeigten die PG-10-Stearat haltigen Zubereitungen keine Instabilitäten, Phasentrennung, Koaleszenz, Ostwald-Reifung oder Veränderung der Tröpfchengröße mit der Zeit oder Aufrahmung. Die Formulierungen, wie sie in den Beispielen offenbart sind, waren mindestens 6 Monate bei Raumtemperatur sowie bei 40°C optisch stabil. Das heißt es wurde keine Phasentrennung oder Phasenabscheidung beobachtet.

Den erfindungsgemäßen Zubereitungen können zusätzliche Emulgatoren zugesetzt werden. Dies ist aber erfindungsgemäß nicht in allen Fällen erforderlich oder zwingend.

Wenn zusätzliche Emulgatoren hinzugefügt werden, sind diese bevorzugt zu wählen aus der Gruppe der Emulgatoren, die bei 25°C fest, pastös oder flüssig und nicht ethoxiliert sind. Besonders bevorzugte zusätzliche Emulgatoren sind zu wählen aus der Gruppe 1,2-Propandiolfettsäureester, Acetoglyceride, acetylierte Mono-/Diglyceride, Alkali- bzw. Ammonium-Seifen, Ammoniumphosphatide, Sorbitanmonoisostearat, C10-C22 Fettsäuren, Cetearyl Sulfat, Cetearylglucoside, Cetylphosphat, Cetylstearylalkohol in Kombination mit Natrium Cetylstearylsulfat, Citroglyceridester, Citronensäuremonoglyceride, Diacetylweinsäuremonoglyceride, Diisostearoyl Polyglyceryl-3 Diisostearat (Isolan PDI), EiLecithin, Emulgator YN (Handelsbezeichnung), Essigsäuremonoglyceride, gemischte Propylenglykol-Glycerinester, Glyceryllaurat, Glycerylmyristat, Glyceryloleat in Kombination mit Propylenglycol, Glycerylstearat, Glycerylstearat SE, Glycerylstearatcitrat, Glycol Distearat, Isostearyldiglycerylsuccinat, Isostearylglycerylether, Kaliumcetylphosphat, Lactoglyceride, lactylierte Mono-/Diglyceride, Lecithin, Methylglucosesesquistearat, Milchsäuremonoglyceride, Mono- und Diglyceride von Speisefettsäuren verestert mit Essigsäure und Weinsäure, Monoglyceridacetat, Monoglyceridcitrat, Monoglyceriddiacetyltartrat, Monoglyceridlactat, Monoglyceridtartrat, Na-,K- und Ca-Salze der Stearoyl-2-lactylmilchsäure, Na-,K- und Ca-stearoyl-2-lactoyl-lactat, Na-,K- und Ca-stearoyl-2-lactyllactat, Na-,K- und Ca-stearoylmilchsäure, Na-Salz der Laurylschwefelsäure, Natriumcetylphosphat, Natriumcetylstearylsulfat, Natriumdodecylsulfat und/oder Dodecylhydrogensulfat, Polyglycerin-3-Diisostearat (LameformTGI), Polyglycerinester der interesterifizierten Ricinolsäure, Polyglycerinfettsäureester, Polyglycerin-Polycrinoleat, Polyglyceryl Dimerate Isostearat, Polyglyceryl Polyricinoleat (Admul WOL 1403), Polyglyceryl-1 Dipolyhydroxystearat (Dehymuls PGPH), Polyglyceryl-2-laurat, Polyglyceryl-2-sesquiisostearat, Polyglyceryl-3 Beeswax (Cera Bellina), Polyglyceryl-3 Cetyl Ether (Chimexane NL), Polyglyceryl-3 Distearat (Cremophor GS 32), Polyglyceryl-3 Methylglucose Distearat (Tego Care 450), Polyglyceryl-3 Oleat, Polyglyceryl-3-methylglycosedistearat, Polyglyceryl-4 Caprat (Polyglycerol Caprate T2010190), Polyglyceryl-4 Diisostearat/Polyhydroxystearat/Sebacate (Isolan GPS), Polyglyceryl-4 Isostearat (Isolan GI 34), Polyoxyethylen-stearinsäureester, Polyoxylstearat-Mono-/Diglycerid, Propylenglycolstearat SE, Propylenglykolfettsäureester, Propylenglykolmono-/di-fettsäureester, Raps-Lecithin, Saccharoglyceride, Saccharoseester von Speisefettsäuren, Soja-Lecithin, Sorbate bsp. Sorbitanmonolaurat (Sorbat 20), Sorbitandicitrat, Sorbitandierucat, Sorbitandihydroxystearat, Sorbitandiisostearat, Sorbitandimaleat, Sorbitandioleat, Sorbitandiricinoleat, Sorbitanditartrat, Sorbitanmonocitrat, Sorbitanmonoerucat, Sorbitanmonohydroxystearat, Sorbitanmonomaleat, Sorbitanmonoricinoleat, Sorbitanmonostearat (Sorbat 60), Sorbitanmonotartrat, Sorbitansesquicitrat, Sorbitansesquierucat, Sorbitansesquihydroxystearat, Sorbitansesquiisostearat, Sorbitansesquimaleat, Sorbitansesquioleat, Sorbitansesquiricinoleat, Sorbitansesquitartrat, Sorbitantricitrat, Sorbitantrierucat, Sorbitantrihydroxystearat, Sorbitantriisostearat, Sorbitanmonooleat, Sorbitantrimaleat, Sorbitantrioleat, Sorbitantriricinoleat, Sorbitantristearat (Sorbat 65), Sorbitantritartrat, Stearinsäure sowie ihrer Salze, Sucroester, Triethylcitrat, Weinsäureglyceride, Weinsäuremonoglyceride und7oder Zuckerfettsäureester, besonders bevorzugt ist Glycerylstearat.

Bevorzugt umfassen die erfindungsgemäßen Zubereitungen keine weiteren zusätzlichen Emulgatoren ausser PG-10-Stearat. Der Anteil an zusätzlichen Emulgatoren sollte somit bevorzugt unter 0,01 Gew.% liegen, bezogen auf die Gesamtmasse der Zubereitung, um als erfindungsgemäß - ohne zusätzlichen Emulgator - zu gelten.

Lecithine ist der klassische Name für eine Gruppe chemischer Verbindungen, die so genannten Phosphatidylcholine. Dabei handelt es sich um Phospholipide, die sich aus Fettsäuren, Glycerin, Phosphorsäure und Cholin zusammensetzen. Lecithine sind Bestandteile der Zellmembran tierischer und pflanzlicher Lebewesen. Sie sind Begleitstoffe in Fetten und Ölen. Sie ermöglichen das Emulgieren von Fetten und Wasser und sind somit wichtige natürliche Tenside (Emulgatoren) für Nahrungs- und Futtermittel. Lecithine sind in der EU als Lebensmittelzusatzstoff (E 322) für Lebensmittel allgemein zugelassen mit Höchstmengenbeschränkung ausschließlich bei Säuglingsnahrung. In der Medizin und in der Kosmetik werden sie auch als Wirkstoff, in der Diätetik als Nahrungsergänzungsmittel genutzt. Den Lecithinen werden neben ihren strukturbildenden Eigenschaften zahlreiche funktionelle Aufgaben zugeschrieben. Sie sind sowohl am anabolen Lipidstoffwechsel als auch am katabolen Fettstoffwechsel aktiv beteiligt.

Lecithine sind daher erfindungsgemäß auszuschließen.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Tenside, Treibgase, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel etc, sofern der Zusatz die geforderten Eigenschaften hinsichtlich mikrobiologischer Stabilität und Hautfreundlichkeit nicht behindert.

Bevorzugte Lipidkomponenten in den erfindungsgemäßen Zubereitungen sind C 12-15 Alkylbenzoat, Isopropylpalmitat, Caprylic/Capric Triglyceride und/oder Octyldodecanol.

Bei der Herstellung der erfindungsgemäßen Zubereitungen zeigt sich ein weiterer unerwarteter Vorteil bei der Verwendung von Polyglyceryl-10-Stearat.

Bei der Emulsionsherstellung muss normalerweise eine Wasser- und eine Fettphase aufgeheizt werden, gemischt und diese Mischung anschließend wieder gekühlt werden. Das bedeutet eine mindestens zweimalige Zugabe von Wärmeenergie. Energetisch ist das ungünstig und ebenso problematisch bei ggf. thermisch labilen Wirkstoffen.

Überraschenderweise ist diese zweimalige Erwärmung durch die Verwendung von Polyglyceryl-10-Stearat als Emulgator nicht notwendig.

Emulsionszubereitungen mit PG-10-Stearat lassen sich im sogenannten cold-cold Verfahren herstellen. Normalerweise schmilzt man die Fettphase auf 80-100°C und heizt parallel die Wasserphase auf 80-100°C auf. Diese beiden heißen Phasen gibt man zusammen und rührt. Anschließend wird homogenisiert. In der Produktion muss dafür häufig gekühlt werden. Dieses Verfahren nennt man hot/hot-Verfahren. Cold/cold-Verfahren heißt also analog, man hat eine Wasserphase bei Raumtemperatur und eine Fettphase bei Raumtemperatur, welche man zusammen gibt und homogenisiert, was energetisch ein großer Vorteil ist.

Die erfindungsgemäßen Zubereitungen sind daher für die Einarbeitung thermisch labiler Wirkstoffe, wie beispielsweise natürlichen Ölen, Wirkstoffe, Vitaminen, Parfum oder auch einzelnen Parfuminhaltsstoffen bevorzugt.

Als thermisch labil bezeichnet man Substanzen, die beim Erwärmen über 50°C sich farblich, geruchlich oder in physikalischen Parametern verändern oder gar sich ganz oder teilweise abbauen.

Die nachfolgenden Beispiele zeigen erfindungsgemäße kosmetische Zubereitungen mit guter mikrobieller Stabilität.

Die angegebenen Anteile sind Gewichtsanteile, jeweils bezogen auf die Gesamtmasse der Zubereitung.

### Beispiele

| INCI | 1 | 2* | 3 | 4 | 5 |
|---|---|---|---|---|---|
| Panthenol | | | | 0.7000 | |
| Piroctone Olamine | | | 0,05 | 0.0500 | 0.1000 |
| Methylisothiazolinone | 0,05 | | | | |
| Glyceryl Caprylate | 0,3 | | 0,3 | | |
| Butylene Glykol | | | | | 0,3 |
| Usnea Barbata(Lichen)Extractta Root Extract, Pulsatilla Koreana Extract, Zanthoxylum Piperitum Fruit Extract | | 0,5 | | | 0,2 |
| Paraffinum Liquidum | | 3.0000 | 3.0000 | | |
| Isopropyl Palmitate | | | | | |
| Caprylic/Capric Triglyceride | 2.3000 | 3.1000 | 3.1000 | | 1.0000 |
| Octyldodecanol | 2.3000 | | | | |
| Cetearyl Alcohol | 1.0000 | | | 2.5000 | 4.0000 |
| Cetyl Alcohol | | 3.0000 | 3.0000 | | |
| Paraffinum Liquidum | | 3.0000 | 3.0000 | | |
| Dimethicone | | 2.8000 | 2.8000 | | 0.5000 |
| Cyclomethicone | | 2.9000 | 2.9000 | | |
| C12-15 Alkyl Benzoate | | | | | 2.0000 |
| Butyrospermum Parkii Butter | | 0.5000 | 0.5000 | | |
| Dicaprylyl Ether | 2.3000 | | | 10.0000 | |
| Dicaprylyl Carbonate | | | | | |
| Glyceryl Stearate | 0.8500 | 1.2000 | 1.2000 | 2.3800 | 2.3800 |
| Glycerin | 6.5000 | 8.6930 | 8.6930 | 8.6930 | 7.5000 |
| Butylene Glycol | | | | 1.0000 | |
| Sodium Hydroxide aq 45% | 0.1500 | 0.0100 | 0.0100 | 0.2500 | 1.0000 |
| Polyglyceryl-10 Stearate | 2.5500 | 1.8000 | 1.8000 | 1.000 | 1.000 |
| Sodium Chloride | 4.0000 | | | | |
| Carbomer | | 0.0200 | 0.0200 | | |
| Acrylic Acid/VP Cross polymer | 0.3000 | | | 0.5000 | 0.7500 |
| Aqua | ad 100 | ad 100 | ad 100 | ad 100 | ad 100 |
| Aqua + Trisodium EDTA | | 1.0000 | 1.0000 | | 1.0000 |
| Ethylhexyl Methoxycinnamate + BHT | | | | 2.0000 | |
| Butyl Methoxydibenzoylmethane | | | | | 2.0000 |
| Phenylbenzimidazole Sulfonic Acid | | | | | 2.0000 |
| Ethylhexyl Salicylate | | | | | 2.0000 |
| Titanium Dioxide + Trimethoxycaprylylsilane | | | | 0.3600 | |
| Octocrylene | | | | | 2.0000 |
| Parfum | | 0.3500 | 0.3500 | | 0.2000 |

| | | | | | |
|---|---|---|---|---|---|
| * Beispiele sind nicht erfindungsgemäß. | | | | | |

| INCI | 6* | 7* | 8* | 9 | 10 |
|---|---|---|---|---|---|
| Ethyllauroylarginate 20% in Glycerin | | 1,8 | 1,8 | 1,8 | 1,8 |
| Phenethylethanol | | | | 0,5 | |
| Phenoxyethanol | | 0,5 | | | 0,5 |
| Caprylyl Glycol | | | 0,5 | 0,5 | |
| Benzylalkohol | | | 0,5 | | |
| Piroctone Olamine | | | | | 0,05 |
| Ethyl Cocoyl Arginate | 0,5 | | | | |
| Paraffinum Liquidum | | 3.0000 | 3.0000 | | |
| Isopropyl Palmitate | | | | | |
| Caprylic/Capric Triglyceride | 2.3000 | 3.1000 | 3.1000 | | 1.0000 |
| Octyldodecanol | 2.3000 | | | | |
| Cetearyl Alcohol | 1.0000 | | | 2.5000 | 4.0000 |
| Cetyl Alcohol | | 3.0000 | 3.0000 | | |
| Paraffinum Liquidum | | 3.0000 | 3.0000 | | |
| Dimethicone | | 2.8000 | 2.8000 | | 0.5000 |
| Cyclomethicone | | 2.9000 | 2.9000 | | |
| C12-15 Alkyl Benzoate | | | | | 2.0000 |
| Butyrospermum Parkii Butter | | 0.5000 | 0.5000 | | |
| Dicaprylyl Ether | 2.3000 | | | 10.0000 | |
| Dicaprylyl Carbonate | | | | | |
| Glyceryl Stearate | 0.8500 | 1.2000 | 1.2000 | 2.3800 | 2.3800 |
| Glycerin | 6.5000 | 8.6930 | 8.6930 | 8.6930 | 7.5000 |
| Butylene Glycol | | | | 1.0000 | |
| Sodium Hydroxide aq 45% | 0.1500 | 0.0100 | 0.0100 | 0.2500 | 1.0000 |
| Polyglyceryl-10 Stearate | 2.5500 | 1.8000 | 1.8000 | 1.000 | 1.000 |
| Sodium Chloride | 4.0000 | | | | |
| Carbomer | | 0.0200 | 0.0200 | | |
| Acrylic Acid/VP Crosspolymer | 0.3000 | | | 0.5000 | 0.7500 |
| Aqua | ad 100 | ad 100 | ad 100 | ad 100 | ad 100 |
| Aqua + Trisodium EDTA | | 1.0000 | 1.0000 | | 1.0000 |
| Butyl Methoxydibenzoylmethane | | 2 | 2 | 2 | 2 |
| Octocrylene | | | | | |
| Phenylbenzimidazol Sulfonsäure | 2 | 2 | 2 | 2 | 2 |
| Diethylamino Hydroxybenzoyl Hexylbenzoate | 2 | | | | |
| Ethylhexylsalicylate | 4 | 4 | 4 | 4 | 4 |
| Diethylhexyl2,6-Naphthalate | | 6 | | | 2.0000 |
| Polyester 8 | | | 6 | | |
| Benzotriazolyl Dodecyl p-Cresol | | | | 6 | |
| Benzophenon-3 | | | | | 6 |
| Parfum | | 0.3500 | 0.3500 | | 0.2000 |

| | | | | | |
|---|---|---|---|---|---|
| * Beispiele sind nicht erfindungsgemäß. | | | | | |

| INCI | 11 | 12 | 13 | 14 | 15 |
|---|---|---|---|---|---|
| Eugenia Caryophyllus (Clove) Floweder Extract | 0,5 | | | | |
| Benzethoniumchlroid | 0,05 | 0,05 | 0,05 | 0,05 | 0,05 |
| Phenoxyethanol | 0,5 | | 0,5 | 0,5 | 0,5 |
| Ethylhexylgylcerin | | 0,5 | | 0,25 | |
| Glyceryl Caprylate | | | 0,3 | | 0,3 |
| Metylpropandiol | | | 3 | | |
| Pentandiol | | 1 | | | |
| Ethanol | | | | 3 | 3 |
| Paraffinum Liquidum | | 3.0000 | 3.0000 | | |
| Isopropyl Palmitate | | | | | |
| Caprylic/Capric Triglyceride | 2.3000 | 3.1000 | 3.1000 | | 1.0000 |
| Octyldodecanol | 2.3000 | | | | |
| Cetearyl Alcohol | 1.0000 | | | 2.5000 | 4.0000 |
| Cetyl Alcohol | | 3.0000 | 3.0000 | | |
| Paraffinum Liquidum | | 3.0000 | 3.0000 | | |
| Dimethicone | | 2.8000 | 2.8000 | | 0.5000 |
| Cyclomethicone | | 2.9000 | 2.9000 | | |
| C12-15 Alkyl Benzoate | 4 | 6 | | 2 | 2.0000 |
| Butyrospermum Parkii Butter | | 0.5000 | 0.5000 | | |
| Dicaprylyl Ether | 2.3000 | | | 10.0000 | |
| Dicaprylyl Carbonate | | | | | |
| Glyceryl Stearate | 0.8500 | 1.2000 | 1.2000 | 2.3800 | 2.3800 |
| Glycerin | 6.5000 | 8.6930 | 8.6930 | 8.6930 | 7.5000 |
| Butylene Glycol | | | | 1.0000 | |
| Sodium Hydroxide aq 45% | 0.1500 | 0.0100 | 0.0100 | 0.2500 | 1.0000 |
| Polyglyceryl-10 Stearate | 2.5500 | 1.8000 | 1.8000 | 1.000 | 1.000 |
| Sodium Chloride | 4.0000 | | | | |
| Carbomer | | 0.0200 | 0.0200 | | |
| Acrylic Acid/VP Cross polymer | 0.3000 | | | 0.5000 | 0.7500 |
| Aqua | ad 100 | ad 100 | ad 100 | ad 100 | ad 100 |
| Aqua + Trisodium EDTA | | 1.0000 | 1.0000 | | 1.0000 |
| Bausteine | | | | 2.0000 | |
| Butyl Methoxydibenzoylmethane | 3 | 4 | 2 | | |
| Octocrylene | 3 | 4 | 2 | | |
| Phenylbenzimidazol Sulfonsäure | 3 | 5 | 2,5 | 2 | 4 |
| Diethylamino Hydroxybenzoyl Hexylbenzoate | | | | 2 | 7 |
| Ethylhexylsalicylate | | | | 3,5 | 4 |
| Parfum | | 0.3500 | 0.3500 | | 0.2000 |

## Patentansprüche

1. Kosmetische oder dermatologische Zubereitung umfassend weniger als 6 Gew.% Polyglyceryl-10-Stearat und ein oder mehrere antimikrobiell wirkende Substanzen im Bereich von mindestens 0,05 Gew.% und maximal 5 Gew.%, jeweils bezogen auf die Gesamtmasse der Zubereitung, wobei die Zubereitung frei von Lecithinen, Parabenen, Formaldehydabspaltern, organischen Halogeniden, ausgenommen_Benzethoniumchlorid, Benzoesäure und ihren Salzen, Formiaten und Teebaumöl ist und O/W-Emulsionszubereitungen 1, 3, 4 und 5 bestehend aus
| | 1 | 3 | 4 | 5 |
|---|---|---|---|---|
| Panthenol | 1,5 | 0 | 0,7 | 0 |
| Butylenglykol | --- | --- | 5 | 10 |
| Propylenglykol | --- | 10 | 5 | --- |
| Benzethoniumchlorid | 1,0 | 0,25 | 0,5 | 0,9 |
| Lauroylethylarginat | 1,0 | 1,5 | 1,0 | 0,5 |
| medizinisches Weissöl | 0 | 6 | 0 | 0 |
| Isopropylpalmitat | 1 | 0 | 0 | 0 |
| Caprylic/Capric Triglycerid | 0 | 3 | 0 | 1,00 |
| Cetearylalkohol | 0 | 0 | 2,5 | 4,00 |
| Cetylalkohol | 0 | 3 | 0 | 0 |
| lineares Silikonöl | 0 | 3 | 0 | 0,50 |
| cyclisches Silikonöl | 0 | 3 | 0 | 0 |
| C12-15 Alkylbenzoat | 0 | 0 | 0 | 2,00 |
| Sheabutter | 0 | 0,5 | 0 | 0 |
| Dicaprylylether | 0 | 0 | 10 | 0 |
| Dicaprylylcarbonat | 2 | 0 | 0 | 0 |
| Glyceryl stearat | 0 | 1,2 | 2,4 | 2,4 |
| Tapiocastärke | 1 | 0 | 0 | 0 |
| Glycerin | 5 | 10 | 8 | 8 |
| Butylenglykol | 0 | 0 | 1 | 0 |
| Zitronensäure | 0,0050 | 0 | 0 | 0 |
| Natronlauge 45% | 0,35 | 0,01 | 0,25 | 1 |
| Polyglyceryl-10 Myristat | 0 | 0 | 0 | 0 |
| Polyglyceryl-10 Stearat | 0,7 | 1,8 | 1 | 1 |
| Polyacrylsäure, Na-Salz (Carbopol 981) | 0,5 | 0,02 | 0 | 0 |
| Acrylic Acid/VP Crosspolymer | 0 | 0 | 0,5 | 0,75 |
| Trinatrium EDTA | 0 | 1 | | 1 |
| Ethylhexylmethoxycinnamat | 0 | 0 | 2 | 0 |
| Butylmethoxydibenzoylmethan | 0 | 0 | 0 | 2 |
| Phenylbenzimidazolesulfonsäure, Natrium-Salz | 0 | 0 | 0 | 2 |
| Ethylhexylsalicylat | 0 | 0 | 0 | 2 |
| Titandioxid, silikongecoated | 0 | 0 | 0,3600 | 0 |
| Octocrylen | 0 | 0 | 0 | 2 |
| Parfum | 0,10 | 0,35 | 0 | 0,20 |
| Wasser | ad 100 | ad 100 | ad 100 | ad 100 |
sowie O/W-Emulsionszubereitungen 46, 48, 49 und 50 bestehend aus
| | 46 | 48 | 49 | 50 |
|---|---|---|---|---|
| Panthenol | 1,5 | 0 | 0,7 | 0 |
| Butylenglykol | --- | --- | 5 | 10 |
| Propylenglykol | --- | 10 | 5 | --- |
| Methylisothiazolinone | --- | --- | 0,06 | --- |
| Benzethoniumchlorid | 2,0 | --- | 0,15 | --- |
| Piroctonolamin | --- | 0,25 | 0,15 | --- |
| Lauroylethylarginat | --- | --- | 0,15 | 1,75 |
| medizinisches Weißöl | 0 | 3 | 0 | 0 |
| Isopropylpalmitat | 1 | 0 | 0 | 0 |
| Caprylic/Capric Triglycerid | 0 | 3 | 0 | 1,00 |
| Cetearylalkohol | 0 | 0 | 2,5 | 4,00 |
| Cetylalkohol | 0 | 3 | 0 | 0 |
| lineares Silikonöl | 0 | 3 | 0 | 0,50 |
| zyklisches Silikonöl | 0 | 3 | 0 | 0 |
| C12-15 Alkylbenzoat | 0 | 0 | 0 | 2,00 |
| Sheabutter | 0 | 0,5 | 0 | 0 |
| Dicaprylylether | 0 | 0 | 10 | 0 |
| Dicaprylylcarbonat | 2 | 0 | 0 | 0 |
| Glyceryl Stearate | 0 | 1,2 | 2,4 | 2,4 |
| Tapiocastärke | 1 | 0 | 0 | 0 |
| Glycerin | 5 | 10 | 8 | 8 |
| Zitronensäure | 0,0050 | 0 | 0 | 0 |
| Natronlauge 45%ig | 0,35 | 0,01 | 0,25 | 1 |
| Polyglyceryl-10 Myristat | 0 | 0 | 0 | 0 |
| Polyglyceryl-10 Stearat | 0,7 | 1,8 | 1 | 1 |
| Polyacrylsäure, Na-Salz | 0,5 | 0,02 | 0 | 0 |
| Acrylic Acid/VP Crosspolymer | 0 | 0 | 0,5 | 0,75 |
| Trinatrium EDTA | 0 | 1 | | 1 |
| Ethylhexylmethoxycinnamat | 0 | 0 | 2 | 0 |
| Butylmethoxydibenzoylmethan | 0 | 0 | 0 | 2 |
| Phenylbenzimidazolesulfonsäure | 0 | 0 | 0 | 2 |
| Ethylhexylsalicylat | 0 | 0 | 0 | 2 |
| Titandioxid+ Trimethoxycaprylylsilan | 0 | 0 | 0,3600 | 0 |
| Octocrylen | 0 | 0 | 0 | 2 |
| Parfum | 0,10 | 0,35 | 0 | 0,20 |
| Wasser | ad 100 | ad 100 | ad 100 | ad 100 |
ausgenommen sind, wobei die Zahlenwerte Gewichtsanteile bezogen auf die Gesamtmasse der Zubereitungen darstellen **dadurch gekennzeichnet, dass** als antimikrobielle Stoffe Piroctone Olamine, Methylisothiazolinone, Phenethylethanol und/oder Benzethoniumchlorid gewählt werden.

2. Zubereitung nach Anspruch 1 umfassend 0,1 bis 2,8 Gew.% Polyglyceryl-10-Stearat, bezogen auf die Gesamtmasse der Zubereitung.

3. Zubereitung nach einem der vorstehenden Ansprüchen umfassend neben Polyglyceryl-10-Stearat keine weiteren Emulgatoren.

4. Zubereitung nach Anspruch 1 oder 2 umfassend neben Polyglyceryl-10-Stearat ein oder mehrere Emulgatoren, die bei 25°C fest, pastös oder flüssig und nicht ethoxiliert sind.

5. Zubereitung nach Anspruch 4 umfassend als zusätzlichen Emulgator Glycerylstearat.

6. Zubereitung nach einem der vorstehenden Ansprüche als O/W-Emulsion.

7. Zubereitung nach einem der Ansprüche 1 bis 5 als Hydrodispersion.

## Claims

1. Cosmetic or dermatological preparation comprising less than 6% by weight of polyglyceryl-10 stearate and one or more antimicrobial reactive substances in the region of at least 0.05% by weight and not more than 5% by weight, based in each case on the total mass of the formulation, where the formulation is free of lecithins, parabens, formaldehyde emitters, organic halides excluding benzethonium chloride, benzoic acid and salts thereof, formates and tea tree oil, and O/W emulsion preparations 1, 3, 4 and 5 consisting of
| | 1 | 3 | 4 | 5 |
|---|---|---|---|---|
| Panthenol | 1.5 | 0 | 0.7 | 0 |
| Butylene glycol | - | - | 5 | 10 |
| Propylene glycol | - | 10 | 5 | - |
| Benzethonium chloride | 1.0 | 0.25 | 0.5 | 0.9 |
| Lauroyl ethyl arginate | 1.0 | 1.5 | 1.0 | 0.5 |
| Medicinal white oil | 0 | 6 | 0 | 0 |
| Isopropyl palmitate | 1 | 0 | 0 | 0 |
| Caprylic/capric triglyceride | 0 | 3 | 0 | 1.00 |
| Cetearyl alcohol | 0 | 0 | 2.5 | 4.00 |
| Cetyl alcohol | 0 | 3 | 0 | 0 |
| Linear silicone oil | 0 | 3 | 0 | 0.50 |
| Cyclic silicone oil | 0 | 3 | 0 | 0 |
| C12-15 alkyl benzoate | 0 | 0 | 0 | 2.00 |
| Shea butter | 0 | 0.5 | 0 | 0 |
| Dicaprylyl ether | 0 | 0 | 10 | 0 |
| Dicaprylyl carbonate | 2 | 0 | 0 | 0 |
| Glyceryl stearate | 0 | 1.2 | 2.4 | 2.4 |
| Tapioca starch | 1 | 0 | 0 | 0 |
| Glycerol | 5 | 10 | 8 | 8 |
| Butylene glycol | 0 | 0 | 1 | 0 |
| Citric acid | 0.0050 | 0 | 0 | 0 |
| Sodium hydroxide solution 45% | 0.35 | 0.01 | 0.25 | 1 |
| Polyglyceryl-10 myristate | 0 | 0 | 0 | 0 |
| Polyglyceryl-10 stearate | 0.7 | 1.8 | 1 | 1 |
| Polyacrylic acid, Na salt (Carbopol 981) | 0.5 | 0.02 | 0 | 0 |
| Acrylic acid/VP crosspolymer | 0 | 0 | 0.5 | 0.75 |
| Trisodium EDTA | 0 | 1 | | 1 |
| Ethylhexyl methoxycinnamate | 0 | 0 | 2 | 0 |
| Butylmethoxydibenzoylmethane | 0 | 0 | 0 | 2 |
| Phenylbenzimidazolesulfonic acid, sodium salt | 0 | 0 | 0 | 2 |
| Ethylhexyl salicylate | 0 | 0 | 0 | 2 |
| Titanium dioxide, silicone-coated | 0 | 0 | 0.3600 | 0 |
| Octocrylene | 0 | 0 | 0 | 2 |
| Perfume | 0.10 | 0.35 | 0 | 0.20 |
| Water | ad 100 | ad 100 | ad 100 | ad 100 |
and O/W emulsion preparations 46, 48, 49 and 50 consisting of
| | 46 | 48 | 49 | 50 |
|---|---|---|---|---|
| Panthenol | 1.5 | 0 | 0.7 | 0 |
| Butylene glycol | - | - | 5 | 10 |
| Propylene glycol | - | 10 | 5 | - |
| Methylisothiazolinones | - | - | 0.06 | - |
| Benzethonium chloride | 2.0 | - | 0.15 | - |
| Piroctone olamine | - | 0.25 | 0.15 | - |
| Lauroyl ethyl arginate | - | - | 0.15 | 1.75 |
| Medicinal white oil | 0 | 3 | 0 | 0 |
| Isopropyl palmitate | 1 | 0 | 0 | 0 |
| Caprylic/capric triglyceride | 0 | 3 | 0 | 1.00 |
| Cetearyl alcohol | 0 | 0 | 2.5 | 4.00 |
| Cetyl alcohol | 0 | 3 | 0 | 0 |
| Linear silicone oil | 0 | 3 | 0 | 0.50 |
| Cyclic silicone oil | 0 | 3 | 0 | 0 |
| C12-15 alkyl benzoate | 0 | 0 | 0 | 2.00 |
| Shea butter | 0 | 0.5 | 0 | 0 |
| Dicaprylyl ether | 0 | 0 | 10 | 0 |
| Dicaprylyl carbonate | 2 | 0 | 0 | 0 |
| Glyceryl stearate | 0 | 1.2 | 2.4 | 2.4 |
| Tapioca starch | 1 | 0 | 0 | 0 |
| Glycerol | 5 | 10 | 8 | 8 |
| Citric acid | 0.0050 | 0 | 0 | 0 |
| Sodium hydroxide solution 45% strength | 0.35 | 0.01 | 0.25 | 1 |
| Polyglyceryl-10 myristate | 0 | 0 | 0 | 0 |
| Polyglyceryl-10 stearate | 0.7 | 1.8 | 1 | 1 |
| Polyacrylic acid, Na salt | 0.5 | 0.02 | 0 | 0 |
| Acrylic acid/VP crosspolymer | 0 | 0 | 0.5 | 0.75 |
| Trisodium EDTA | 0 | 1 | | 1 |
| Ethylhexyl methoxycinnamate | 0 | 0 | 2 | 0 |
| Butylmethoxydibenzoylmethane | 0 | 0 | 0 | 2 |
| Phenylbenzimidazolesulfonic acid | 0 | 0 | 0 | 2 |
| Ethylhexyl salicylate | 0 | 0 | 0 | 2 |
| Titanium dioxide + tri meth oxycaprylylsilane | 0 | 0 | 0.3600 | 0 |
| Octocrylene | 0 | 0 | 0 | 2 |
| Perfume | 0.10 | 0.35 | 0 | 0.20 |
| Water | ad 100 | ad 100 | ad 100 | ad 100 |
are excluded, where the numerical values are fractions by weight, based on the total mass of the preparations, **characterized in that** the antimicrobial substances chosen are piroctone olamine, methylisothiazolinone, phenethylethanol and/or benzethonium chloride.

2. Preparation according to Claim 1, comprising 0.1 to 2.8% by weight of polyglyceryl-10 stearate, based on the total mass of the preparation.

3. Preparation according to one of the preceding claims, comprising no further emulsifiers besides polyglycerol-10 stearate.

4. Preparation according to Claim 1 or 2, comprising, besides polyglyceryl-10 stearate, one or more emulsifiers which, at 25°C, are solid, pasty or liquid and not ethoxylated.

5. Preparation according to Claim 4, comprising glyceryl stearate as additional emulsifier.

6. Preparation according to one of the preceding claims as O/W emulsion.

7. Preparation according to one of Claims 1 to 5 as hydrodispersion.

## Revendications

1. Préparation cosmétique ou dermatologique comprenant moins de 6 % en poids de stéarate de polyglycéryl-10 et une ou plusieurs substances à effet antimicrobien dans la plage d'au moins 0,05 % en poids et d'au plus 5 % en poids, à chaque fois par rapport à la masse totale de la préparation, la préparation étant exempte de lécithines, de parabènes, de cliveurs de formaldéhyde, d'halogénures organiques, à l'exception du chlorure de benzéthonium, d'acide benzoïque et ses sels, de formiates et d'huile d'arbre à thé, et les préparations en émulsion H/E 1, 3, 4 et 5 constituées par :
| | 1 | 3 | 4 | 5 |
|---|---|---|---|---|
| Panthénol | 1,5 | 0 | 0,7 | 0 |
| Butylène glycol | --- | --- | 5 | 10 |
| Propylène glycol | --- | 10 | 5 | --- |
| Chlorure de benzéthonium | 1,0 | 0,25 | 0,5 | 0,9 |
| Arginate de lauroyléthyle | 1,0 | 1,5 | 1,0 | 0,5 |
| Huile blanche médicale | 0 | 6 | 0 | 0 |
| Palmitate d'isopropyle | 1 | 0 | 0 | 0 |
| Triglycéride caprylique/caprique | 0 | 3 | 0 | 1,00 |
| Alcool cétéarylique | 0 | 0 | 2,5 | 4,00 |
| Alcool cétylique | 0 | 3 | 0 | 0 |
| Huile de silicone linéaire | 0 | 3 | 0 | 0,50 |
| Huile de silicone cyclique | 0 | 3 | 0 | 0 |
| Benzoate d'alkyle en C12-15 | 0 | 0 | 0 | 2,00 |
| Beurre de karité | 0 | 0,5 | 0 | 0 |
| Ether dicaprylique | 0 | 0 | 10 | 0 |
| Carbonate de dicaprylyle | 2 | 0 | 0 | 0 |
| Stéarate de glycéryle | 0 | 1,2 | 2,4 | 2,4 |
| Amidon de tapioca | 1 | 0 | 0 | 0 |
| Glycérine | 5 | 10 | 8 | 8 |
| Butylène glycol | 0 | 0 | 1 | 0 |
| Acide citrique | 0,0050 | 0 | 0 | 0 |
| Soude caustique 45 % | 0,35 | 0,01 | 0,25 | 1 |
| Myristate de polyglycéryl-10 | 0 | 0 | 0 | 0 |
| Stéarate de polyglycéryl-10 | 0,7 | 1,8 | 1 | 1 |
| Acide polyacrylique, sel Na (Carbopol 981) | 0,5 | 0,02 | 0 | 0 |
| Polymère réticulé d'acide acrylique/VP | 0 | 0 | 0,5 | 0,75 |
| EDTA trisodique | 0 | 1 | | 1 |
| Méthoxycinnamate d'éthylhexyle | 0 | 0 | 2 | 0 |
| Butylméthoxydibenzoylméthane | 0 | 0 | 0 | 2 |
| Acide phénylbenzimidazole-sulfonique, sel de sodium | 0 | 0 | 0 | 2 |
| Salicylate d'éthylhexyle | 0 | 0 | 0 | 2 |
| Dioxyde de titane, revêtu de silicone | 0 | 0 | 0,3600 | 0 |
| Octocrylène | 0 | 0 | 0 | 2 |
| Parfum | 0,10 | 0,35 | 0 | 0,20 |
| Eau | Qsp 100 | Qsp 100 | Qsp 100 | Qsp 100 |
ainsi que les préparations en émulsion H/E 46, 48, 49 et 50 constituées par :
| | 46 | 48 | 49 | 50 |
|---|---|---|---|---|
| Panthénol | 1,5 | 0 | 0,7 | 0 |
| Butylène glycol | --- | --- | 5 | 10 |
| Propylène glycol | --- | 10 | 5 | --- |
| Méthylisothiazolinone | --- | --- | 0,06 | --- |
| Chlorure de benzéthonium | 2,0 | --- | 0,15 | --- |
| Piroctonolamine | --- | 0,25 | 0,15 | --- |
| Arginate de lauroyléthyle | --- | --- | 0,15 | 1,75 |
| Huile blanche médicale | 0 | 3 | 0 | 0 |
| Palmitate d'isopropyle | 1 | 0 | 0 | 0 |
| Triglycéride caprylique/caprique | 0 | 3 | 0 | 1,00 |
| Alcool cétéarylique | 0 | 0 | 2,5 | 4,00 |
| Alcool cétylique | 0 | 3 | 0 | 0 |
| Huile de silicone linéaire | 0 | 3 | 0 | 0,50 |
| Huile de silicone cyclique | 0 | 3 | 0 | 0 |
| Benzoate d'alkyle en C12-15 | 0 | 0 | 0 | 2,00 |
| Beurre de karité | 0 | 0,5 | 0 | 0 |
| Ether dicaprylique | 0 | 0 | 10 | 0 |
| Carbonate de dicaprylyle | 2 | 0 | 0 | 0 |
| Stéarate de glycéryle | 0 | 1,2 | 2,4 | 2,4 |
| Amidon de tapioca | 1 | 0 | 0 | 0 |
| Glycérine | 5 | 10 | 8 | 8 |
| Acide citrique | 0,0050 | 0 | 0 | 0 |
| Soude caustique 45 % | 0,35 | 0,01 | 0,25 | 1 |
| Myristate de polyglycéryl-10 | 0 | 0 | 0 | 0 |
| Stéarate de polyglycéryl-10 | 0,7 | 1,8 | 1 | 1 |
| Acide polyacrylique, sel Na | 0,5 | 0,02 | 0 | 0 |
| Polymère réticulé d'acide acrylique/VP | 0 | 0 | 0,5 | 0,75 |
| EDTA trisodique | 0 | 1 | | 1 |
| Méthoxycinnamate d'éthylhexyle | 0 | 0 | 2 | 0 |
| Butylméthoxydibenzoylméthane | 0 | 0 | 0 | 2 |
| Acide phénylbenzimidazole-sulfonique | 0 | 0 | 0 | 2 |
| Salicylate d'éthylhexyle | 0 | 0 | 0 | 2 |
| Dioxyde de titane + triméthoxycaprylylsilane | 0 | 0 | 0,3600 | 0 |
| Octocrylène | 0 | 0 | 0 | 2 |
| Parfum | 0,10 | 0,35 | 0 | 0,20 |
| Eau | Qsp 100 | Qsp 100 | Qsp 100 | Qsp 100 |
étant exclues, les valeurs numériques étant des proportions en poids relatives à la masse totale des préparations, **caractérisée en ce que** de la piroctone olamine, de la méthylisothiazolinone, du phénéthyléthanol et/ou du chlorure de benzéthonium sont choisis en tant que substances antimicrobiennes.

2. Préparation selon la revendication 1, comprenant 0,1 à 2,8 % en poids de stéarate de polyglycéryl-10, par rapport à la masse totale de la préparation.

3. Préparation selon l'une quelconque des revendications précédentes, ne comprenant pas d'autres émulsifiants en plus du stéarate de polyglycéryl-10.

4. Préparation selon la revendication 1 ou 2, comprenant un ou plusieurs émulsifiants en plus du stéarate de polyglycéryl-10, qui sont solides, pâteux ou liquides à 25 °C, et non éthoxylés.

5. Préparation selon la revendication 4, comprenant du stéarate de glycéryle en tant qu'émulsifiant supplémentaire.

6. Préparation selon l'une quelconque des revendications précédentes, sous la forme d'une émulsion H/E.

7. Préparation selon l'une quelconque des revendications 1 à 5, sous la forme d'une hydrodispersion.
